# EUROPEAN PATENT APPLICATION

(11) **EP 4 785 848 A1**
(43) Date of publication of application: **05.08.2026**
(21) Application number: 24872414.8
(22) Date of filing: 26.09.2024
(51) Int. Cl.: A61B 1/005, A61B 1/00, A61B 1/04, C09J 11/04, C09J 11/06, C09J 163/00

(54) **ADHESIVE FOR ENDOSCOPE, ENDOSCOPE, AND METHOD FOR PRODUCING SAME**

(30) Priority: 29.09.2023 JP 2023170304
(71) Applicant: FUJIFILM Corporation, Tokyo 106-8620 (JP)
(72) Inventor: FURUKAWA, Kazushi, Ashigarakami-gun, Kanagawa 258-8577 (JP)
(74) Representative: HGF
(86) International application number: PCT/JP2024/034467
(87) International publication number: WO 2025/070629

(57) **Abstract**

An adhesive for an endoscope, an endoscope, and a manufacturing method of the endoscope, the adhesive for an endoscope including:
(A) an epoxy resin,
(B) a polyamine compound having two or more unsubstituted amino groups, and
(C) a fluorescent organic compound having a maximum fluorescence wavelength of 380 to 525 nm in an N,N-dimethylformamide solution.

## Description

### BACKGROUND OF THE INVENTION

### 1. Field of the Invention

The present invention relates to an adhesive for an endoscope, an endoscope, and a manufacturing method of the endoscope.

### 2. Description of the Related Art

In manufacturing an endoscope for observing an internal body cavity of a human body, an inside of a digestive tract such as an esophagus, a stomach, and an intestine, an inside of a respiratory tract such as a trachea and a bronchus, and the like, an adhesive is used to assemble fine components and fix the components or to seal a void.

Among the adhesives, an epoxy-based adhesive is excellent in workability, and an adhesive, a heat resistance, a moisture resistance, and the like of a cured product thereof are also excellent, so that the epoxy-based adhesive is also used for bonding constituent members of the endoscope. Since the endoscope is repeatedly used over a long period of time, it is required that the fixed state of the endoscope member by the adhesive can be sufficiently maintained even in a case where the endoscope is repeatedly used for a long period of time. That is, it is required to have durability such that the fixed state can be sufficiently maintained even in a case where the endoscope is immersed in a disinfectant or subjected to a sterilization treatment.

For example, WO2022/070809A describes an adhesive for an endoscope including an epoxy resin including at least one of a bisphenol A-type epoxy resin, a bisphenol F-type epoxy resin, or a phenol novolac-type epoxy resin, a curing component (curing agent) of the epoxy resin including at least one of a phosphorus-containing compound, a polythiol compound, a dicyandiamide compound, a phenol compound, a polyamine compound having an unsubstituted amino group, an acid anhydride compound, or an imidazole compound, and a specific radical scavenger. According to WO2022/070809A, the adhesive is excellent in heat resistance in a state (in a state of a cured product) where the adhesive is used for fixing the endoscope member, and is also excellent in durability against a strong sterilization treatment such as an ozone water.

### SUMMARY OF THE INVENTION

In addition to the durability against the sterilization treatment, it is required that the adhesive for an endoscope sufficiently enters a narrow gap between the fine components during the assembly to reliably fix the components. In a case where the adhesive does not sufficiently enter the gap between the fine components (hereinafter, also referred to as "insufficient entry of the adhesive into the gap"), the assembly is defective, and the necessary functions such as strength cannot be sufficiently exhibited.

Therefore, it is necessary to check whether or not there is the insufficient entry of the adhesive into the gap during the assembly of the endoscope. However, in order to suppress the influence on the image during the image observation using the endoscope, the components fixed by the adhesive and the adhesive itself are often black, and it is difficult to determine the filling state of the adhesive into the gap by color (appearance).

The present inventors considered that, by adding a fluorescent organic compound to the adhesive, irradiating the fluorescent organic compound with excitation light, and causing the adhesive to emit light, even in a case where the amount of the fluorescent organic compound added is a small amount that is not expected to affect the observation using the endoscope, due to the black surroundings, it is possible to easily check for insufficient entry of the adhesive into the gap, and thus it is possible to reduce the adhesive failure during the assembly.

However, as a result of actual studies, it has been found that, even in a case where the amount of the fluorescent organic compound added is set to be a small amount, the fluorescence emission caused by the light included in the observation light source during white light observation using the endoscope undesirably affects the image quality of the observation image.

An object of the present invention is to provide an adhesive for an endoscope that can easily determine whether or not there is an insufficient entry of the adhesive into a gap and can suppress the influence of an adhesive component on an observation image in white light observation using the endoscope.

Another object of the present invention is to provide an endoscope including a cured product of the adhesive for an endoscope as a fixing material of a constituent member (component) of the endoscope, in which the influence of an adhesive component on an observation image in white light observation is suppressed. Another object of the present invention is to provide a manufacturing method of the endoscope using the adhesive for an endoscope.

The foregoing objects of the present invention have been achieved by the following means.
<1> An adhesive for an endoscope, comprising:
   (A) an epoxy resin
   (B) a polyamine compound having two or more unsubstituted amino groups
   (C) a fluorescent organic compound having a maximum fluorescence wavelength of 380 to 525 nm in an N,N-dimethylformamide solution
<2> The adhesive for an endoscope according to <1>, in which the component (B) includes a polyether polyamine compound.
<3> The adhesive for an endoscope according to <1> or <2>, in which a content of the component (C) is 0.005 to 0.500 parts by mass with respect to 100 parts by mass of the component (A).
<4> The adhesive for an endoscope according to any one of <1> to <3>, further comprising: 5 to 30 parts by mass of an inorganic amphoteric ion exchanger as a component (D) with respect to 100 parts by mass of the component (A).
<5> An endoscope including a member fixed by the adhesive for an endoscope according to any one of <1> to <4>.
<6> A manufacturing method of an endoscope, including fixing a member by using the adhesive for an endoscope according to any one of <1> to <4>.

In the description of the present invention, the expression "to" is used to mean that numerical values described before and after "to" are included as a lower limit value and an upper limit value, respectively.

In the adhesive for an endoscope according to an aspect of the present invention, each of the components (the components (A) to (C) and the optional component) may be used alone or in combination of two or more thereof unless otherwise specified.

In the present invention, with regard to a substituent (the same applies to a linking group) in which whether it is substituted or unsubstituted is not specified, within a range not impairing the desired effect, it means that the group may have an optional substituent. Examples of such a substituent include a substituent selected from a substituent group T described later. The same shall be applied to a compound which is not specified in the present specification regarding whether to be substituted or unsubstituted.

In the present specification, in a case where the number of carbon atoms of a certain group is defined, the number of carbon atoms means the number of carbon atoms of the entire group. That is, in a case where this group has a form that further has a substituent, it means the total number of carbon atoms, to which the number of carbon atoms of this substituent is included.

The adhesive for an endoscope according to the aspect of the present invention can easily determine whether or not there is an insufficient entry of the adhesive into a gap, and can suppress the influence of an adhesive component on an observation image in white light observation using the endoscope. In addition, the endoscope according to the aspect of the present invention includes a cured product of the adhesive for an endoscope as a fixing material of a constituent member (component) of the endoscope, in which the influence of an adhesive component on an observation image in white light observation is suppressed. Further, according to the manufacturing method of an endoscope according to the present invention, it is possible to easily determine whether or not there is an insufficient entry of the adhesive into a gap, and it is possible to obtain an endoscope in which the influence of an adhesive component on an observation image in white light observation is suppressed.

### BRIEF DESCRIPTION OF THE DRAWINGS

FIG. 1 is an external view showing a configuration of an endoscope according to an embodiment of the present invention.
FIG. 2 is a partial cross-sectional view showing a configuration of an insertion part of the endoscope shown in FIG. 1.
FIG. 3 is an external perspective view of a distal end part of the insertion part.
FIG. 4 is a partially cutout cross-sectional view of the distal end part. The cross-hatching indicating the sections of the lenses and prisms has been omitted.
FIG. 5 is an explanatory view of an evaluation sample S1 used in Evaluation 1 of Examples.
FIG. 6 is an explanatory view of an evaluation sample S2 used in Evaluation 2 of Examples.

### DESCRIPTION OF THE PREFERRED EMBODIMENTS

### [Adhesive for endoscope]

The adhesive for an endoscope according to the embodiment of the present invention (hereinafter, also referred to as an "adhesive of the present invention") includes the following components (A) to (C).
Component (A): an epoxy resin;
Component (B): a polyamine compound having two or more unsubstituted amino groups; and
Component (C): a fluorescent organic compound having a maximum fluorescence wavelength of 380 to 525 nm in an N,N-dimethylformamide solution.

The component (A) is a main agent of the adhesive, and the component (B) is a curing agent that reacts with the epoxy resin to cure the adhesive. The adhesive according to the embodiment of the present invention contains the fluorescent organic compound of the component (C) in addition to the main agent and the curing agent.

A form of the adhesive according to the embodiment of the present invention is not particularly limited as long as it contains the above-described respective components. For example, the adhesive for an endoscope according to the embodiment of the present invention may be in a form of containing a mixture of the components (A) to (C) (one-part type), or may contain the components (A) to (C) in a state where a part of the components (A) to (C) is separated from the other components (two-part type). In addition, the adhesive for an endoscope according to the embodiment of the present invention may contain the components (A) to (C) in a state where the components (A) to (C) are separated from each other (three-part type). Any of these forms are included in the adhesive according to the embodiment of the present invention. However, it is preferable that the fluorescent organic compound of the component (C) is in a state of being uniformly dispersed in the adhesive and the cured product in a state where the components (A) to (C) are mixed (before curing) and in a state of the cured product after curing.

In the present specification, in a case where the content of each component in the adhesive is described or the content of each component in the adhesive is defined in the present invention, in the form of the two-part type, the three-part type, or the like, the mixing of the components (A) to (C) at the time of use means that the content of each component in the mixture satisfies a desired content described later. That is, in a state where the components are separated from each other, the content of each of the components (A) to (C) does not need to satisfy the content described in the present specification or the content defined in the present invention. That is, in the form of the two-part type, the three-part type, or the like, it means that the content described in the present specification or the content defined in the present invention is satisfied at the time of mixing the components (A) to (C) at the time of use.

In a case where the adhesive for an endoscope according to the embodiment of the present invention is in the form of the one-part type, or in a case where components that can react with each other are mixed in the form of the two-part type or the like (for example, in a case where the epoxy resin of the component (A) and the curing agent of the component (B) are mixed), it is preferable that the adhesive is stored at a low temperature to a level at which the reaction virtually does not occur, in order to maintain a state where the components are stably maintained without causing or sufficiently suppressing the reaction between the components. For example, the adhesive can be stored at 0°C or lower, may be stored at -10°C or lower, may be stored at -20°C or lower, may be stored at -30°C or lower, may be stored at -40°C or lower, or may be stored at -50°C or lower. In addition, the adhesive can be stored in a light-shielded manner as necessary.

The adhesive according to the embodiment of the present invention, in addition to the components (A) to (C), may include, for example, a solvent, a plasticizer, a curing accelerator, an adhesion improver (such as a silane coupling agent), a surfactant, a colorant (such as a pigment, a dye, excluding the fluorescent organic compound), a weatherproofing agent, an antioxidant, a heat stabilizer, a lubricant, an antistatic agent, a mold release agent, a conductive agent, a viscosity modifier, a filler (such as silica, calcium carbonate), a thixotropy imparting agent, a diluent, and a flame retardant, and the like, to the extent that the effects of the present invention are not impaired.

The cured product obtained by curing the adhesive according to the embodiment of the present invention makes it possible to easily determine the presence or absence of insufficient entry of the adhesive into the gaps, and to suppress the influence of the adhesive component (fluorescent organic compound) on an observation image in white light observation using an endoscope.

The adhesive of the present invention is suitable for fixing various members (endoscope constituent members) constituting the endoscope. That is, the adhesive of the present invention is suitably used for bonding (joining) and fixing the endoscope constituent member to another constituent member of the endoscope. The adhesive used for fixing the endoscope constituent member is cured to form a cured product that constitutes an adhesive portion of the endoscope. Among these, the adhesive of the present invention can be suitably used for fixing the endoscope constituent member of the endoscope that performs the white light observation using the white light.

A member to be fixed by the adhesive according to the embodiment of the present invention is not particularly limited, and examples thereof include a metal member, a glass member, and a resin member. The "fix" of the endoscope constituent member is performed by bonding the endoscope constituent member to another member (support member) constituting the endoscope. The support member may be a tube wall or the like of the endoscope or a non-movable member fixed to the tube wall or the like, or may be a member such as a tube in which a relative position in the endoscope can be moved. In addition, the term "fixing" in the present invention is used to mean that a space between the constituting member of the endoscope and the support member in which the constituting member of the endoscope is incorporated is filled with the cured product of the adhesive, that is, sealed.

Each component constituting the adhesive according to the embodiment of the present invention will be described below.

### <(A) Epoxy resin>

The adhesive of the present invention includes an epoxy resin as the component (A). It is preferable that the epoxy resin includes at least one of a bisphenol A-type epoxy resin, a bisphenol F-type epoxy resin, or a phenol novolac-type epoxy resin. The adhesive of the present invention may include one kind of epoxy resin selected from a bisphenol A-type epoxy resin, a bisphenol F-type epoxy resin, or a phenol novolac-type epoxy resin, or may include two or more kinds thereof.

A proportion of the total amount of the bisphenol A-type epoxy resin, the bisphenol F-type epoxy resin, and the phenol novolac-type epoxy resin to the total amount of the epoxy resin included in the adhesive of the present invention is preferably 70% by mass or more, more preferably 80% by mass or more, and still more preferably 90% by mass or more. The epoxy resin included in the adhesive according to the embodiment of the present invention is particularly preferably at least one of a bisphenol A-type epoxy resin, a bisphenol F-type epoxy resin, or a phenol novolac-type epoxy resin.

An epoxy equivalent of the epoxy resin contained in the adhesive according to the embodiment of the present invention is preferably 10 to 1,000, more preferably 50 to 500, still more preferably 80 to 400, and particularly preferably 100 to 300. The epoxy resin contained in the adhesive according to the embodiment of the present invention usually has two or more epoxy groups in one molecule.

The epoxy equivalent is a value obtained by dividing the molecular weight of the epoxy compound by the number of moles of epoxy groups included in the epoxy compound.

The bisphenol A-type epoxy resin which can be used in the adhesive according to the embodiment of the present invention is not particularly limited, and any bisphenol A-type epoxy resin commonly used as a main agent of an epoxy-based adhesive can be widely used. Preferred specific examples thereof include bisphenol A diglycidyl ethers (jER825, jER828, and jER834 (all trade names, manufactured by Mitsubishi Chemical Corporation)) and bisphenol A propoxylate diglycidyl ethers (for example, manufactured by Sigma-Aldrich Co. LLC).

The bisphenol F-type epoxy resin which can be used in the adhesive according to the embodiment of the present invention is not particularly limited, and any bisphenol F-type epoxy resin commonly used as a main agent of an epoxy-based adhesive can be widely used. Preferred specific examples thereof include bisphenol F diglycidyl ether (trade name: EPICLON 830, manufactured by DIC Corporation).

The phenol novolac type epoxy resin usable in the adhesive according to the embodiment of the present invention is not particularly limited, and a resin generally used as a main agent of an epoxy adhesive can be widely used. Such a phenol novolac type epoxy resin is commercially available, for example, by the product number 406775 from Sigma-Aldrich Co. LLC.

A content of the epoxy resin contained in the adhesive according to the embodiment of the present invention can be 5% to 99% by mass, more preferably 10% to 98% by mass, still more preferably 20% to 97% by mass, and particularly preferably 30% to 96% by mass.

### <(B) Polyamine compound having two or more unsubstituted amino groups>

The adhesive according to the embodiment of the present invention contains a polyamine compound having two or more unsubstituted amino groups (-NH₂) (hereinafter, the polyamine compound having two or more unsubstituted amino groups is also simply referred to as a "polyamine compound") as the component (B). The adhesive according to the embodiment of the present invention may contain only one type of the component (B), or a combination of two or more types of the component (B).

The polyamine compound is more preferably a primary polyamine compound (polyamine compound in which all amino groups are unsubstituted amino groups).

The number of unsubstituted amino groups in one molecule of the polyamine compound is preferably 2 to 10, more preferably 2 to 8, still more preferably 2 to 6, even more preferably 2 to 4, and particularly preferably 2 or 3. Among these, at least one selected from a diamine compound or a triamine compound can be suitably used.

An active hydrogen equivalent (equivalent of active hydrogen contained in an amino group) of the polyamine compound is preferably 25 to 2,000, more preferably 25 to 550, still more preferably 25 to 200, even more preferably 25 to 100, even still more preferably 25 to 85, and particularly preferably 27 to 70.

The active hydrogen equivalent is a value obtained by dividing the molecular weight of the polyamine compound by the number of moles of active hydrogen of the amino group in the polyamine compound (means molecular weight per one active hydrogen of the amino group in the polyamine compound). Examples of the amino group having active hydrogen include an unsubstituted amino group and a primary amino group.

A molecular weight of the polyamine compound is preferably 100 to 6,000, more preferably 100 to 3,000, still more preferably 100 to 1,000, and particularly preferably 100 to 500. In a case where the polyamine compound is a polymer (for example, in a case of having a polyoxyalkylene group described later), the molecular weight is a number average molecular weight.

In the polyamine compound, it is preferable that two or more unsubstituted amino groups are bonded to each other through a chain-like aliphatic hydrocarbon group, an alicyclic hydrocarbon group, an aromatic hydrocarbon group, a heterocyclic group, or a group obtained by combining these groups. In the group obtained by combining these groups, a heteroatom (an atom other than a carbon atom, preferably an oxygen atom or a nitrogen atom, and more preferably an oxygen atom) such as an oxygen atom, a nitrogen atom, or a sulfur atom may be included between carbon-carbon bonds, and it is preferable that the heteroatom is included.

The group that bonds the two or more unsubstituted amino groups is preferably a chain-like aliphatic hydrocarbon group, a combination of a chain-like aliphatic hydrocarbon group and an aromatic hydrocarbon group, a combination of a chain-like aliphatic hydrocarbon group and an alicyclic hydrocarbon group, a group having a heteroatom between carbon-carbon bonds in a combination of two or more chain-like aliphatic hydrocarbon groups, a group having a heteroatom between carbon-carbon bonds in a combination of a chain-like aliphatic hydrocarbon group and an aromatic hydrocarbon group, or a group having a heteroatom between carbon-carbon bonds in a combination of a chain-like aliphatic hydrocarbon group and an alicyclic hydrocarbon group, more preferably a group having a heteroatom between carbon-carbon bonds in a combination of two or more chain-like aliphatic hydrocarbon groups or a group having a heteroatom between carbon-carbon bonds in a combination of a chain-like aliphatic hydrocarbon group and an aromatic hydrocarbon group, and still more preferably a group having a heteroatom between carbon-carbon bonds in a combination of two or more chain-like aliphatic hydrocarbon groups.

The "chain-like aliphatic hydrocarbon group" may have a branch, and in a case where the group that bonds the two or more unsubstituted amino groups is the chain-like aliphatic hydrocarbon group, the chain-like aliphatic hydrocarbon group preferably has a branched structure.

As the "combination of a chain-like aliphatic hydrocarbon group and an aromatic hydrocarbon group", for example, "chain-like aliphatic hydrocarbon group-aromatic hydrocarbon group-chain-like aliphatic hydrocarbon group" is preferable.

As the "combination of a chain-like aliphatic hydrocarbon group and an alicyclic hydrocarbon group", for example, "chain-like aliphatic hydrocarbon group-alicyclic hydrocarbon group" and "alicyclic hydrocarbon group-chain-like aliphatic hydrocarbon group-alicyclic hydrocarbon group" are exemplified.

As the "group having a heteroatom between carbon-carbon bonds in two or more chain-like aliphatic hydrocarbon groups", a group having a polyether structure described later is exemplified.

As the "group having a heteroatom between carbon-carbon bonds in a combination of a chain-like aliphatic hydrocarbon group and an aromatic hydrocarbon group", "[chain-like aliphatic hydrocarbon group-aromatic hydrocarbon group-chain-like aliphatic hydrocarbon group-nitrogen atom-chain-like aliphatic hydrocarbon group-nitrogen atom]-chain-like aliphatic hydrocarbon group-aromatic hydrocarbon group-chain-like aliphatic hydrocarbon group" is exemplified. The structure represented by [ ] means a repeating unit, and for example, the number of repetitions is 1 to 20.

The number of carbon atoms in the chain-like aliphatic hydrocarbon group is preferably 2 to 50 and more preferably 2 to 12. The chain-like aliphatic hydrocarbon group is preferably an alkylene group, and specific examples of the alkylene group include methylene, ethylene, propylene, hexamethylene, 2,4,4-trimethylhexamethylene, and dodecamethylene.

The number of carbon atoms in the alicyclic hydrocarbon group is preferably 4 to 50, more preferably 4 to 12, and still more preferably 6 to 12. The cyclic hydrocarbon group is preferably a cycloalkylene group, and specific examples of the cycloalkylene group include cyclobutylene, cyclopentylene, and cyclohexylene.

Examples of the aromatic hydrocarbon ring constituting the aromatic hydrocarbon group include a benzene ring and a fused ring of two or more benzene rings, and specific examples of the fused ring include a fluorene ring and an acenaphthene ring. Among these aromatic hydrocarbon rings, a benzene ring is preferable.

From the viewpoint of making it easier to determine whether or not there is an insufficient entry of the adhesive into a gap and further suppressing the influence on the observation image in white light observation using the endoscope, the polyamine compound is preferably a polyether polyamine compound.

The polyether polyamine compound means a compound having a polyether structure and two or more unsubstituted amino groups. The polyether structure is preferably a repeating structure of a group consisting of a group selected from the above-described chain-like aliphatic hydrocarbon group, the alicyclic hydrocarbon group, the aromatic hydrocarbon group, or the heterocyclic group and -O-, and preferably has a repeating structure of a group consisting of the above-described chain-like aliphatic hydrocarbon group and -O-, that is, a polyoxyalkylene structure.

The alkylene group constituting the polyoxyalkylene structure may be a linear alkylene group or an alkylene group having a branch. In addition, the number of carbon atoms in the alkylene group constituting the polyoxyalkylene structure is preferably 1 to 10, more preferably 2 to 6, and still more preferably 2 to 4.

The polyoxyalkylene structure is more preferably a polyoxyethylene structure or a polyoxypropylene structure.

In a case where the polyamine compound of the component (B) has a polyoxyalkylene structure, a plurality of oxyalkylene groups constituting the polyoxyalkylene structure may be the same or different from each other. In addition, an average number of repetitions of the oxyalkylene group in the polyoxyalkylene structure (in a case of having two or more kinds of polyoxyalkylene structures, it means a total of the number of repetitions) is preferably 2 to 1,000, more preferably 2 to 500, still more preferably 2 to 100, even more preferably 2 to 50, particularly preferably 2 to 35, and among these, preferably 2 to 25. The polyamine compound of the component (B) may have a plurality of polyoxyalkylene structures.

Preferred specific examples of the polyamine compound that can be used in the present invention are shown below. The number in parentheses is an average number of repetitions of the repeating unit in the parentheses.

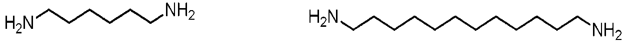

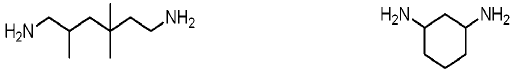

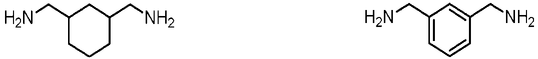

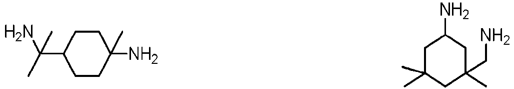

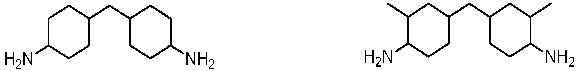

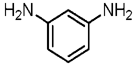

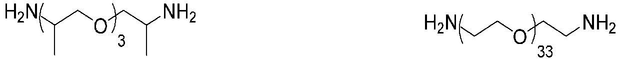

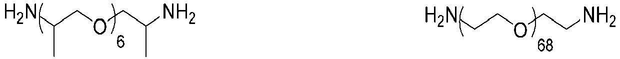

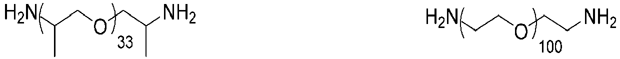

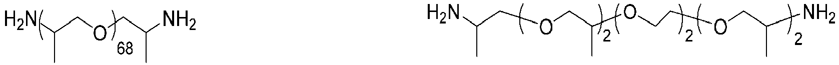

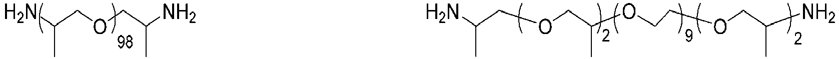

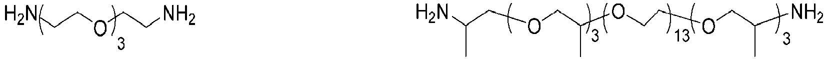

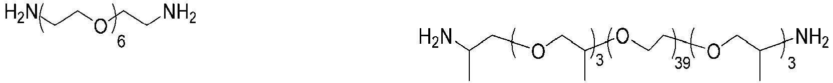

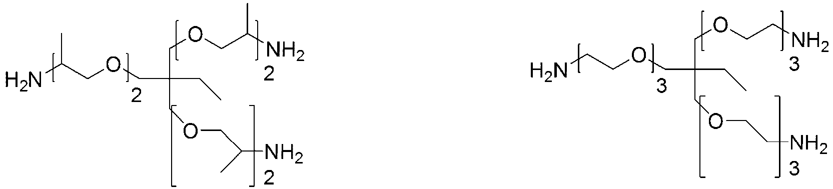

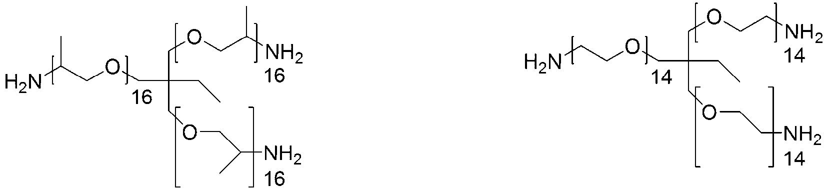

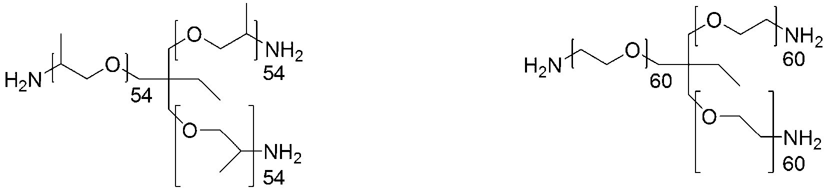

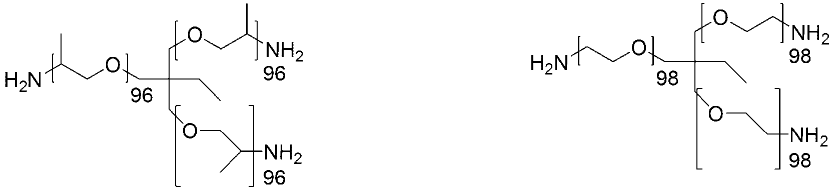

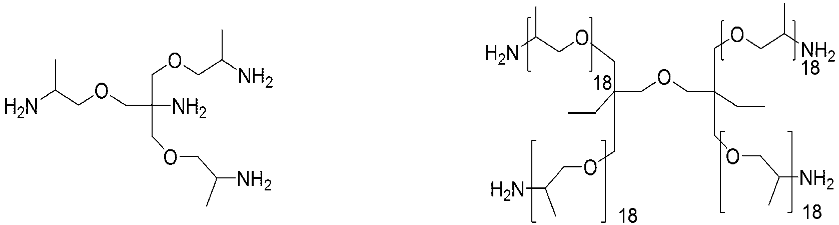

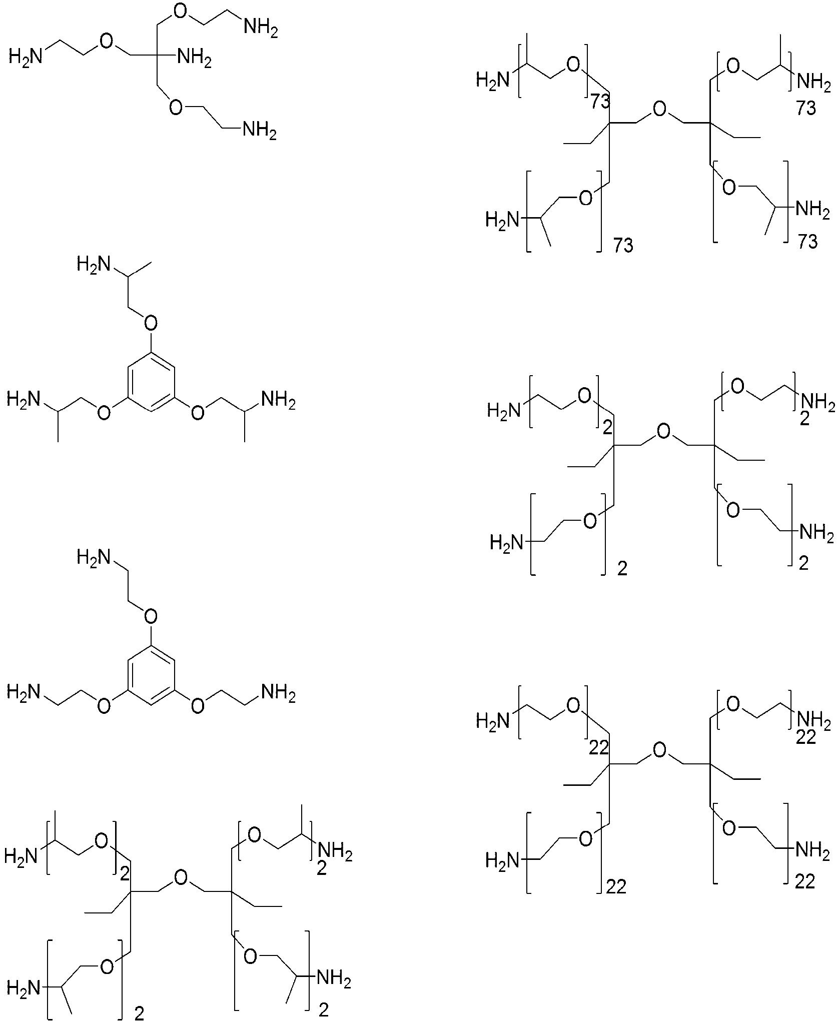

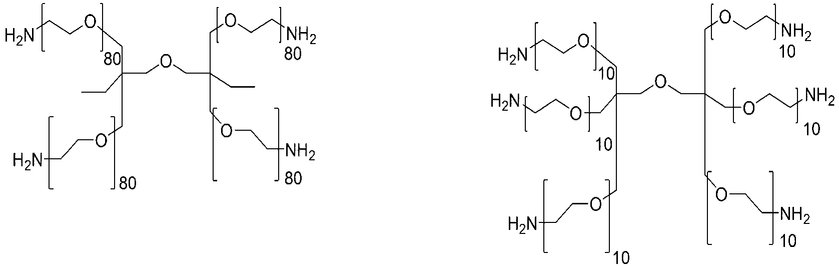

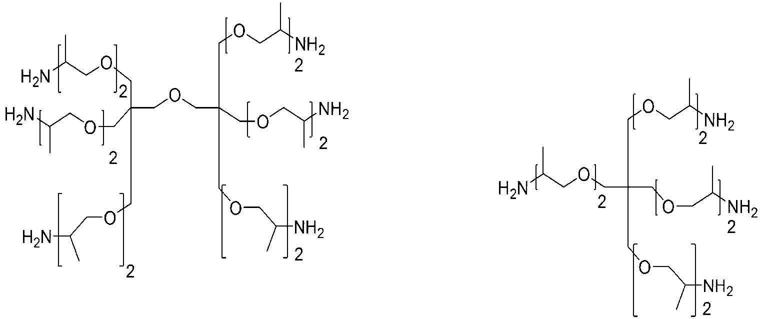

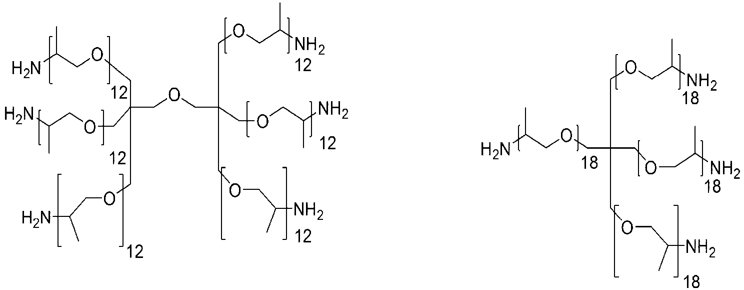

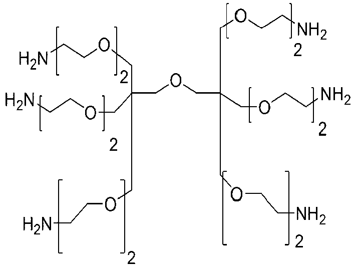

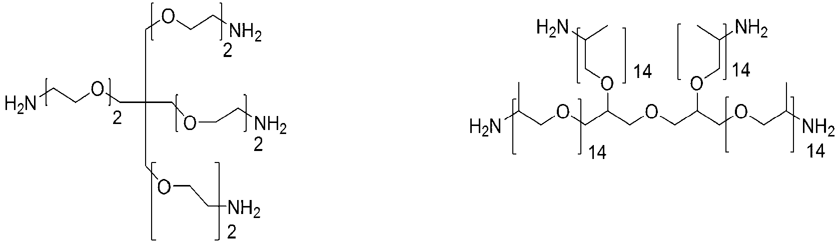

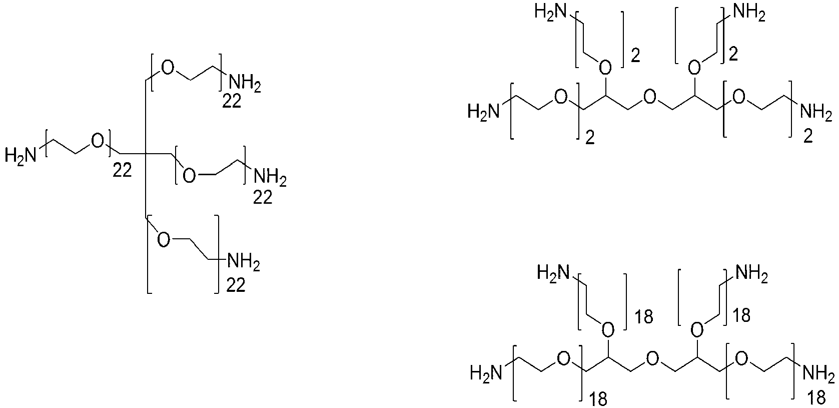

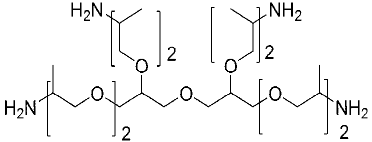

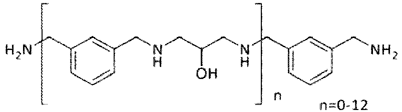

The polyamine compound can be synthesized by a conventional method. In addition, a commercially available product may be used as the polyamine compound.

A content of the polyamine compound of the component (B) in the adhesive according to the embodiment of the present invention is not particularly limited, and can be appropriately prepared in accordance with the reaction between the component (A) and the component (B) or the like.

In the adhesive according to the embodiment of the present invention, a content of the polyamine compound of the component (B) can be appropriately set in consideration of the active hydrogen equivalent or the like.

For example, the content of the polyamine compound of the component (B) can be set to 5 to 100 parts by mass, preferably 8 to 75 parts by mass, and more preferably 10 to 50 parts by mass with respect to 100 parts by mass of the epoxy resin as the component (A). In addition, it is preferable that an active hydrogen equivalent of the polyamine compound (active hydrogen equivalent/epoxy equivalent) with respect to the epoxy equivalent of the epoxy resin as the component (A) is set to 0.05 to 1.5, more preferably 0.10 to 1.2, and still more preferably 0.15 to 1.0.

### <(C) Fluorescent organic compound having maximum fluorescence wavelength of 380 to 525 nm in N,N-dimethylformamide solution>

The adhesive according to the embodiment of the present invention contains a fluorescent organic compound having a maximum fluorescence wavelength of 380 to 525 nm in an N,N-dimethylformamide (DMF) solution.

In the present invention, the "maximum fluorescence wavelength in a DMF solution" means a wavelength at which the maximum fluorescence intensity is exhibited in a fluorescence spectrum obtained by exciting the fluorescent organic compound using a spectrophotofluorometer such as RF-6000 (trade name, manufactured by Shimadzu Corporation) under a condition of a measurement temperature of 25°C in a DMF solution having a concentration of 1 ppm of the fluorescent organic compound.

The maximum fluorescence wavelength of the fluorescent organic compound of the component (C) in the DMF solution is preferably 400 to 525 nm and more preferably 420 to 525 nm.

The fluorescent organic compound of the component (C) does not have two or more unsubstituted amino groups.

The fluorescent organic compound of the component (C) can be used without particular limitation as long as it is a fluorescent organic compound satisfying the above-described maximum fluorescence wavelength, and examples thereof include a coumarin compound, a thiophene compound, a stilbene compound, and a monomethine diaryl compound (a compound in which an aromatic ring group (which may be an aryl group or a heteroaryl group) is bonded to both ends of a monomethine chain represented by -CH=CH-). However, the stilbene compound is excluded), a fluorescein compound, a naphthalene compound, a perylene compound, and a rhodamine compound.

Among these, as the fluorescent organic compound of the component (C), a fluorescent organic compound having a benzoxazole structure is preferable, and a bis(benzoxazolyl)thiophene compound, a 1,2-bis(benzoxazolylphenyl)ethene compound (a compound in which a phenyl ring of a stilbene compound has a benzoxazolyl group as a substituent), or a bis(benzoxazolyl)ethylene compound (a compound in which an aryl group in the monomethine diaryl compound is a benzoxazolyl group) is more preferable. The benzoxazole structure may have a substituent, and examples of the substituent include an alkyl group.

The benzoxazole structure of the fluorescent organic compound of the component (C) preferably interacts with a benzene ring in the bisphenol A-type epoxy resin, the bisphenol F-type epoxy resin, or the phenol novolac-type epoxy resin, which are included in the epoxy resin of the component (A), to increase the solubility of the component (C) in the component (A), and to make the component (C) more uniformly present in the adhesive for an endoscope according to the embodiment of the present invention and the cured product thereof.

A molecular weight of the fluorescent organic compound of the component (C) can be, for example, 150 to 1,000, and from the viewpoint of more easily determining whether or not there is an insufficient entry of the adhesive into a gap and further suppressing the influence of the adhesive component on the observation image in white light observation using the endoscope, it is preferably 170 to 800, more preferably 190 to 600, and still more preferably 220 to 500.

In the adhesive for an endoscope according to the embodiment of the present invention, a content of the fluorescent organic compound of the component (C) can be appropriately set in a range in which it is possible to easily determine whether or not there is an insufficient entry of the adhesive into a gap and it is possible to suppress the influence of the adhesive component on the observation image in white light observation using the endoscope.

For example, the content of the fluorescent organic compound of the component (C) can be set to 0.005 to 0.500 parts by mass, preferably 0.007 to 0.300 parts by mass, and more preferably 0.010 to 0.200 parts by mass with respect to 100 parts by mass of the epoxy resin as the component (A).

### <(D) Inorganic amphoteric ion exchanger>

The adhesive for an endoscope according to the embodiment of the present invention preferably contains an inorganic amphoteric ion exchanger.

The "inorganic amphoteric ion exchanger" means a metal-containing inorganic compound that exhibits an ion exchange phenomenon for both cations and anions. That is, the inorganic amphoteric ion exchanger used in the present invention is a metal-containing inorganic compound that can release cations and anions contained in the inorganic amphoteric ion exchanger itself into a solution in a case of being brought into contact with an aqueous solution of salts, and instead take in cations and anions in the solution into the inorganic amphoteric ion exchanger.

The inorganic amphoteric ion exchanger that can be used in the adhesive for an endoscope according to the embodiment of the present invention is not particularly limited, and is preferably an inorganic compound including at least one of a bismuth (Bi) atom, an antimony (Sb) atom, a zirconium (Zr) atom, a magnesium (Mg) atom, or an aluminum (Al) atom, and more preferably an inorganic compound including two or three of the above-described atoms.

Examples of the combination of two of the above-described atoms include a combination of an Sb atom and a Bi atom, and a combination of a Zr atom and a Bi atom. Specific examples of the inorganic amphoteric ion exchanger including a combination of an Sb atom and a Bi atom include IXE-600 and IXE-633 (both are trade names (manufactured by Toagosei Co., Ltd.)). Specific examples of the inorganic amphoteric ion exchanger including a combination of a Zr atom and a Bi atom include IXE-6107, IXE-6136, and IXEPLAS-B1 (all are trade names (manufactured by Toagosei Co., Ltd.)).

Examples of the combination of three of the above-described atoms include a combination of a Zr atom, an Mg atom, and an Al atom. Specific examples of the inorganic amphoteric ion exchanger including a combination of a Zr atom, an Mg atom, and an Al atom include IXEPLAS-A1 and IXEPLAS-A2 (both are trade names (manufactured by Toagosei Co., Ltd.)). It is noted that "IXE" and "IXEPLAS" are registered trademarks.

In the adhesive for an endoscope according to the embodiment of the present invention, a shape of the inorganic amphoteric ion exchanger is not particularly limited, and for example, may be particulate or irregular, and is preferably particulate. A median diameter (D50) of the inorganic amphoteric ion exchanger is, for example, preferably 0.1 to 10 µm, more preferably 0.1 to 5 µm, and still more preferably 0.1 to 3 µm.

The median diameter (D50) can be determined using a particle size distribution measuring device (for example, LA-950V2 (trade name) manufactured by HORIBA, Ltd.) based on "particle diameter analysis-laser diffraction method" defined in JIS Z 8825-1:2013.

A content of the inorganic amphoteric ion exchanger in the adhesive for an endoscope according to the embodiment of the present invention is not particularly limited, and for example, is preferably 5 to 30 parts by mass, more preferably 7 to 30 parts by mass, still more preferably 10 to 30 parts by mass, even more preferably 15 to 30 parts by mass, and particularly preferably 20 to 30 parts by mass with respect to 100 parts by mass of the epoxy resin as the component (A).

### <(E) Inorganic filler>

The adhesive for an endoscope according to the embodiment of the present invention may contain an inorganic filler as the component (E). The inorganic filler of the component (E) functions as, for example, a viscosity regulator, a filler, a colorant, and the like.

Examples of the inorganic filler include so-called inorganic solid fillers such as alumina (aluminum oxide), magnesia (magnesium oxide), titanium oxide (titanium white), aluminum hydroxide, barium titanate, zinc oxide, silica (including crystalline silica (silicon oxide) and fused silica (silicon oxide)), metal nanoparticles, and glass fibers; layer silicates such as talc, clay, mica, smectite, kaolin minerals, mica clay, and vermiculite; metal powders such as silver powder and copper powder; and nitrides such as aluminum nitride, boron nitride, silicon nitride, and gallium nitride, which function as a viscosity adjuster, a filler, and the like.

In addition, as the inorganic filler, silicon carbide, carbon black, graphite, carbon fiber, carbon nanotube, and the like can also be used, which function as a black colorant.

It is also preferable that the inorganic filler is subjected to a surface treatment. Such a surface treatment is not particularly limited. Examples thereof include a surface treatment with a silane compound. In the present invention, the silane compound means a compound having a structure in which at least one organic group is bonded to Si. It is more preferably SiR₄ (R₄ is an organic group). As the silane compound, a silane coupling agent, a silazane, or a silicone compound (polysiloxane) is preferable. Such a surface treatment method can be carried out by a conventional method. For example, paragraphs [0090] to [0101] of JP2018-195964A can be referred to.

### The inorganic filler can be commercially obtained.

Examples thereof include, as a commercially available product of the above-described alumina, DAM-70, DAM-45, DAM-07, DAM-05, DAW-45, DAW-05, DAW-03, and ASF-P-20 (all trade names, manufactured by Denka Company Limited), AL-43-KT, AL-47-H, AL-47-1, AL-160SG-3, AL-43-BE, AS-30, AS-40, AS-50, AS-400, CB-P02, and CB-P05 (all trade names, manufactured by Showa Denko K.K.), A31, A31B, A32, A33F, A41A, A43A, MM-22, MM-26, MM-P, MM-23B, LS-110F, LS-130, LS-210, LS-242C, LS-250, and AHP300 (all trade names, manufactured by Nippon Light Metal Co., Ltd.), AA-03, AA-04, AA-05, AA-07, AA-2, AA-5, AA-10, and AA-18 (all trade names, manufactured by Sumitomo Chemical Co., Ltd.), and AEROXIDE Alu C, AEROXIDE Alu C805, and AEROXIDE Alu 65 (all trade names, manufactured by Nippon Aerosil Co., Ltd.).

Examples of the commercially available product of the above-described titanium oxide include G-1, G-10, F-2, F-4, and F-6 (all trade names, manufactured by Showa Denko K.K.), TAF-520, TAF-500, TAF-1500, TM-1, TA-100C, and TA-100CT (all trade names, manufactured by Fuji Titanium Industry Co., Ltd.), MT-01, MT-10EX, MT-05, MT-100S, MT-100TV, MT-100Z, MT-150EX, MT-100AQ, MT-100WP, MT-100SA, MT-100HD, MT-300HD, MT-500SA, MT-600SA, and MT-700HD (all trade names, manufactured by Teika Co., Ltd.), TTO-51 (A), TTO-51 (C), TTO-55 (A), TTO-55 (B), TTO-55 (C), TTO-55 (D), TTO-S-1, TTO-S-2, TTO-S-3, TTO-S-4, MPT-136, and TTO-V-3 (all trade names, manufactured by ISHIHARA SANGYO KAISHA, LTD.), and AEROXIDE NKT90 (trade name, manufactured by Nippon Aerosil Co., Ltd.).

Examples of the commercially available product of the above-described aluminum hydroxide include B-309 and B-309 (both trade names, manufactured by Tomoe Engineering Co., Ltd.), and BA173, BA103, B703, B1403, BF013, BE033, BX103, and BX043 (all trade names, manufactured by Nippon Light Metal Co., Ltd.).

Examples of the commercially available product of the above-described layer silicate include NANO ACE D-1000, NANO ACE D-800, MICRO ACE SG-95, MICRO ACE P-8, and MICRO ACE P-6 (all trade names of talc, manufactured by Nippon Talc Co., Ltd.), FH104, FH105, FL108, FG106, MG115, FH104S, and ML112S (all trade names of talc, manufactured by Fuji Talc Co., Ltd.), Y-1800, TM-10, A-11, and SJ-005 (all trade names of mica, manufactured by Yamaguchi Mica Co., Ltd.), and KUNIBIS-110 (trade name, manufactured by Kunimine Industries Co., Ltd.).

Examples of the commercially available product of the above-described barium titanate include BT-H9DX, HF-9, HF-37N, HF-90D, HF-120D, and HT-F (all trade names, manufactured by Kyoritsu Material Co., Ltd.), BT-100 and HPBT series (all trade names, manufactured by Fuji Titanium Industry Co., Ltd.), BT series (trade name, manufactured by SAKAI CHEMICAL INDUSTRY Co., Ltd.), and PULCERAM BT (trade name, manufactured by Nippon Chemical Industrial Co., Ltd.).

Examples of the commercially available product of the above-described zinc oxide include FINEX-30, FINEX-30W-LP2, FINEX-50, FINEX-50S-LP2, and XZ-100F (all trade names, manufactured by SAKAI CHEMICAL INDUSTRY Co., Ltd.), FZO-50 (trade name, manufactured by ISHIHARA SANGYO KAISHA, LTD.), and MZ-300, MZ-306X, MZY-505S, MZ-506X, and MZ-510HPSX (all trade names, manufactured by Teika Co., Ltd.).

Examples of the commercially available product of the above-described glass fiber include CS6SK-406, CS13C-897, CS3PC-455, and CS3LCP-256 (all trade names, manufactured by NITTOBO Co., Ltd.), ECS03-615, ECS03-650, EFDE50-01, and EFDE50-31 (all trade names, manufactured by Central Glass Co., Ltd.), ACS6H-103 and ACS6S-750 (all trade names, manufactured by Nippon Electric Glass Co., Ltd.), and CF0027, CF0093, CF0018, and CF0033 (all trade names, manufactured by Nippon Frit Co., Ltd.).

Examples of the commercially available product of the above-described metal powder include AG3 and AG4 which are spherical silver powders (both trade names, manufactured by Dowa High-Tech Materials Co., Ltd.), FA5 and FA2 which are flake silver powders (both trade names, manufactured by Dowa High-Tech Materials Co., Ltd.), SPQ03R, SPN05N, SPN08S, and Q03R (all trade names of silver powders, manufactured by Mitsui Mining & Smelting Co., Ltd.), AY-6010 and AY-6080 (both trade names of silver powders, manufactured by Tanaka Kikinzoku Kogyo K.K.), ASP-100 (trade name of silver powder, manufactured by Aida Chemical Industries Co., Ltd.), Ag coat powder AG/SP (trade name of silver powder, manufactured by Mitsubishi Materials Electronic Chemicals Co., Ltd.), MA-O015K, MA-O02K, and MA-O025K (all trade names of copper powders, manufactured by Mitsui Mining & Smelting Co., Ltd.), electrolytic copper powder #52-C and #6 (manufactured by JX Nippon Mining & Metals Corporation), 10% Ag coat Cu-HWQ (trade name of copper powder, manufactured by FUKUDA METAL FOIL & POWDER Co., Ltd.), Type-A and Type-B (both trade names of copper powders, manufactured by Dowa Electronics Co., Ltd.), and UCP-030 (trade name of copper powder, manufactured by Sumitomo Metal Mining Co., Ltd.).

Examples of the commercially available product of the above-described nitride include H grade, E grade, and H-T grade (all trade names of aluminum nitride, manufactured by Tokuyama Corporation), TOYAL TecFiller TFS-A05P and TOYAL TecFiller TFZ-A02P (both trade names of aluminum nitride, manufactured by TOYO ALUMINUM K.K.), ALN020BF, ALN050BF, ALN020AF, ALN050AF, and ALN020SF (all trade names of aluminum nitride, manufactured by Tomoe Engineering Co., Ltd.), FAN-f05 and FAN-f30 (both trade names of aluminum nitride, manufactured by Furukawa Electric Co., Ltd.), DENKA BORON NITRIDE SGP, DENKA BORON NITRIDE MGP, DENKA BORON NITRIDE GP, DENKA BORON NITRIDE HGP, DENKA BORON NITRIDE SP-2, and DENKA BORON NITRIDE SGPS (all trade names of boron nitride, manufactured by Denka Company Limited), UHP-S1, UHP-1K, UHP-2, and UHP-EX (all trade names of boron nitride, manufactured by Showa Denko K.K.), and SN-9, SN-9S, SN-9FWS, SN-F1, and SN-F2 (all trade names of silicon nitride, manufactured by Denka Company Limited).

Examples of the commercially available product of the above-described silicon carbide include GMF-H type, GMF-H2 type, and GMF-LC type (all trade names, manufactured by Pacific Random Co., Ltd.), and HSC1200, HSC1000, HSC059, HSC059I, and HSC007 (all trade names, manufactured by Tomoe Engineering Co., Ltd.).

Examples of the commercially available product of the above-described silica include SICILIA (manufactured by Fuji Silysia Chemical Ltd.), AEROSIL R972, AEROSIL R104, AEROSIL R202, AEROSIL 805, AEROSIL R812, AEROSIL RX200, AEROSIL R9200, AEROSIL NAX50, AEROSIL 200, and AEROSIL R7200 (all trade names, manufactured by Nippon Aerosil Co., Ltd.), LEOSHEEL series (manufactured by Tokuyama Corporation), CMC-12, VX-S, and VX-SR (all trade names of crystalline silica, manufactured by Tatsumori Ltd.), FB-3SDC, FB-3SDX, SFP-30M, SFP-20M, SFP-30MHE, SFP-130MC, and UFP-30 (all trade names of fused silica, manufactured by Denka Company Limited), and EXCELICA series (trade name of fused silica, manufactured by Tokuyama Corporation).

Examples of the commercially available product of the above-described carbon fiber, carbon black, graphite, and carbon nanotube include Torayca Milled Fiber MLD-30 and Torayca Milled Fiber MLD-300 (both trade names of carbon fibers, manufactured by Toray Industries, Inc.), CFMP-30X and CFMP-150X (both trade names of carbon fibers, manufactured by Nippon Polymer Co., Ltd.), #1000 (trade name of carbon black, manufactured by Mitsubishi Chemical Corporation), XN-100 and HC-600 (both trade names of graphite, manufactured by Nippon Graphite Fiber Co., Ltd.), DENKA BLACK (trade name of carbon black, manufactured by Denka Company Limited), and SWeNT SG65, SWeNT SGi, IsoNanoTubes-M, IsoNanoTubes-S, PureTubes, Pyrograf PR-25-XT-PS, and PR-25XT-LHT (all trade names of carbon nanotubes, manufactured by Sigma-Aldrich Co., LLC.).

The inorganic filler preferably includes at least one of silica (preferably fumed silica), titanium oxide, alumina, or a layer silicate, which functions as a viscosity adjuster, and more preferably includes silica. In addition, from the viewpoint of improving chemical resistance, it is preferable that these are subjected to a surface treatment and hydrophobized. It is more preferable that at least an alkyl group is introduced onto the surface of the inorganic filler by the surface treatment, that is, an alkyl-modified inorganic filler. Among these, surface-treated silica (preferably alkyl-modified silica particles) is suitable.

In addition, the inorganic filler preferably includes at least one of silicon carbide, carbon black, graphite, carbon fiber, or carbon nanotube, which functions as a black colorant, and more preferably includes carbon black.

A median diameter (D50) of the inorganic filler used in the present invention on a volume basis is preferably 1 to 10,000 nm, more preferably 3 to 5,000 nm, still more preferably 5 to 2,000 nm, even more preferably 5 to 1,000 nm, even still more preferably 6 to 500 nm, even further more preferably 7 to 200 nm, and particularly preferably 8 to 100 nm. In addition, in a case of silica, titanium oxide, and alumina, from the viewpoint of chemical resistance of the cured product, the median diameter (D50) on a volume basis is preferably 1 to 500 nm, more preferably 5 to 200 nm, still more preferably 6 to 100 nm, and particularly preferably 7 to 50 nm.

The median diameter (D50) of the inorganic filler on a volume basis is measured by the following method.

The inorganic filler is added to methanol such that the content thereof is 0.5% by mass, and the mixture is subjected to ultrasonic waves for 10 minutes to disperse the inorganic filler. The particle size distribution of the inorganic filler treated in this way is measured by a laser diffraction scattering type particle size distribution measuring device (manufactured by HORIBA, Ltd., trade name: LA950V2), and the median diameter (D50) on a volume basis is determined. The median diameter corresponds to a cumulative 50% in a case where the particle size distribution is represented as a cumulative distribution.

In the adhesive for an endoscope according to the embodiment of the present invention, from the viewpoint of viscosity of the adhesive, a content of the inorganic filler of the component (E) is preferably 4 to 20% by mass, more preferably 4 to 15% by mass, and still more preferably 4 to 12% by mass.

In addition, in the adhesive for an endoscope according to the embodiment of the present invention, a content of the black colorant is preferably 1 to 15% by mass, more preferably 1.5 to 10% by mass, and still more preferably 2 to 8% by mass.

In the adhesive for an endoscope according to the embodiment of the present invention, a content of the inorganic filler of the component (E) can be set to 1 to 50 parts by mass, and is preferably 4 to 20 parts by mass with respect to 100 parts by mass of the epoxy resin as the component (A).

In addition, in the adhesive for an endoscope according to the embodiment of the present invention, a content of the black colorant can be set to 0.25 to 37.5 parts by mass, and is preferably 1 to 13.3 parts by mass with respect to 100 parts by mass of the epoxy resin as the component (A).

It is preferable that the inorganic filler of the component (E) is in a state of being uniformly dispersed in the adhesive in a state where the components (A) to (C) are mixed (before curing).

### - Substituent group T -

In the present invention, the preferred substituents include those selected from the following substituent group T.

In the present invention, in a case where the substituent is simply described as a substituent, the description of the corresponding substituent in the substituent group T can be referred to and applied. For example, in a case where only "alkyl group" is simply described, the description of "alkyl group" in the substituent group T can be referred to and applied. The same applies to other substituents other than the "alkyl group".

In addition, in the present invention, examples of the substituent which may be contained in a certain substituent such as an "alkyl group" include a substituent selected from the following substituent group T. In addition, in a case where a certain substituent such as an "alkyl group" has a substituent and further has a substituent, examples of the substituent which is contained in the certain substituent such as an "alkyl group" include a substituent obtained by combining two or more substituents selected from the following substituent group T.

Further, in the present specification, in a case where an alkyl group is described separately from a cyclic (cyclo)alkyl group, the alkyl group is meant to include a linear alkyl group and a branched alkyl group. On the other hand, in a case where an alkyl group is not described separately from a cyclic alkyl group, and unless otherwise specified, the alkyl group is meant to include a linear alkyl group, a branched alkyl group, and a cycloalkyl group. The same applies to groups (alkoxy group, alkylthio group, alkenyloxy group, and the like) containing a group capable of having a cyclic structure (alkyl group, alkenyl group, alkynyl group, and the like) and compounds containing a group capable of having a cyclic structure. In a case where a group is capable of forming a cyclic skeleton, the lower limit of the number of atoms of the group forming the cyclic skeleton is 3 or more and preferably 5 or more, regardless of the lower limit of the number of atoms specifically described below for the group that can adopt this structure.

In the description of the substituent group T below, a group having a linear or branched structure and a group having a cyclic structure are sometimes described separately, for example, an alkyl group and a cycloalkyl group, in order to clearly distinguish between the group having a linear or branched structure and the group having a cyclic structure.

The groups included in the substituent group T include the following groups.

Examples thereof include an alkyl group (preferably having 1 to 30 carbon atoms, more preferably having 1 to 20 carbon atoms, still more preferably having 1 to 12 carbon atoms, even still more preferably having 1 to 8 carbon atoms, yet even still more preferably having 1 to 6 carbon atoms, and particularly preferably having 1 to 3 carbon atoms), an alkenyl group (preferably having 2 to 30 carbon atoms, more preferably having 2 to 20 carbon atoms, still more preferably having 2 to 12 carbon atoms, even still more preferably having 2 to 6 carbon atoms, and yet even still more preferably having 2 to 4 carbon atoms), an alkynyl group (preferably having 2 to 30 carbon atoms, more preferably having 2 to 20 carbon atoms, still more preferably having 2 to 12 carbon atoms, even still more preferably having 2 to 6 carbon atoms, and yet even still more preferably having 2 to 4 carbon atoms), a cycloalkyl group (preferably having 3 to 20 carbon atoms), a cycloalkenyl group (preferably having 5 to 20 carbon atoms), an aryl group (may be a monocyclic group or a fused ring group (preferably a fused group of 2 to 6 rings). In a case of a fused ring group, the fused ring group consists of a 5-membered to 7-membered ring. Preferably having 6 to 40 carbon atoms, more preferably 6 to 30 carbon atoms, still more preferably 6 to 26 carbon atoms, and particularly preferably 6 to 10 carbon atoms), a heterocycle group (having at least one nitrogen atom, an oxygen atom, a sulfur atom, a phosphorus atom, a silicon atom, or selenium atom as a ring-constituting atom; may be a monocyclic ring or a fused ring (preferably a fused group in which 2 to 6 rings). In a case of a monocyclic group, the monocyclic ring is preferably a 5-membered to 7-membered ring and more preferably a 5-membered or 6-membered ring. The heterocycle group preferably has 2 to 40 carbon atoms and more preferably 2 to 20 carbon atoms. The heterocyclic group includes an aromatic heterocyclic group (heteroaryl group) and an aliphatic heterocyclic group (aliphatic heterocyclic group)), an alkoxy group (preferably having 1 to 20 carbon atoms and more preferably having 1 to 12 carbon atoms), an alkenyloxy group (preferably having 2 to 20 carbon atoms and more preferably having 2 to 12 carbon atoms), an alkynyloxy group (preferably having 2 to 20 carbon atoms and more preferably having 2 to 12 carbon atoms), a cycloalkyloxy group (preferably having 3 to 20 carbon atoms), an aryloxy group (preferably having 6 to 40 carbon atoms, more preferably having 6 to 26 carbon atoms, and still more preferably having 6 to 14 carbon atoms), a heterocyclic oxy group (preferably having 2 to 20 carbon atoms).

An alkoxycarbonyl group (preferably having 2 to 20 carbon atoms), a cycloalkoxycarbonyl group (preferably having 4 to 20 carbon atoms), an aryloxycarbonyl group (preferably having 6 to 20 carbon atoms), an amino group (preferably having 0 to 20 carbon atoms; including an unsubstituted amino group (-NH₂), a (mono- or di-) alkylamino group, a (mono- or di-) alkenylamino group, a (mono- or di-) alkynylamino group, a (mono- or di-) cycloalkylamino group, a (mono- or di-) cycloalkenylamino group, a (mono- or di-) arylamino group, and a (mono- or di-) heterocyclic amino group. Each of the above groups substituting the unsubstituted amino group has the same definition as the corresponding group in the substituent group T), a sulfamoyl group (preferably having 0 to 20 carbon atoms; the sulfamoyl group is preferably an alkyl, cycloalkyl, or aryl sulfamoyl group), an acyl group (preferably having 1 to 20 carbon atoms, and more preferably having 2 to 15 carbon atoms), an acyloxy group (preferably having 1 to 20 carbon atoms), a carbamoyl group (preferably having 1 to 20 carbon atoms; the carbamoyl group is preferably an alkyl, cycloalkyl, or aryl carbamoyl group).

An acylamino group (preferably having 1 to 20 carbon atoms), a sulfonamide group (preferably having 0 to 20 carbon atoms; and preferably an alkyl sulfonamide group, a cycloalkyl sulfonamide group, or an aryl sulfonamide group), an alkylthio group (preferably having 1 to 20 carbon atoms and more preferably having 1 to 12 carbon atoms), a cycloalkylthio group (preferably having 3 to 20 carbon atoms), an arylthio group (preferably having 6 to 40 carbon atoms, more preferably having 6 to 26 carbon atoms, and still more preferably having 6 to 14 carbon atoms), a heterocyclic thio group (preferably having 2 to 20 carbon atoms), an alkyl, cycloalkyl, or aryl sulfonyl group (preferably having 1 to 20 carbon atoms).

A silyl group (preferably having 1 to 30 carbon atoms and more preferably 1 to 20 carbon atoms, and preferably substituted with an alkyl, aryl, alkoxy, or aryloxy), a silyloxy group (preferably having 1 to 20 carbon atoms and preferably substituted with an alkyl, aryl, alkoxy, or aryloxy), a hydroxy group, a cyano group, a nitro group, a halogen atom (for example, a fluorine atom, a chlorine atom, a bromine atom or an iodine atom), an oxygen atom (specifically replacing >CH₂ constituting a ring with >C=O), a carboxy group (-CO₂H), a phosphono group [-PO(OH)₂], a phosphoryl group [-O-PO(OH)₂], a sulfo group (-SO₃H), a boronic acid group [-B(OH)₂], an onium group (including an ammonio group including a cyclic ammonio group, a sulfonio group, and a phosphonio group, and preferably having 0 to 30 carbon atoms and more preferably having 1 to 20 carbon atoms), a sulfanyl group (-SH), an amino acid residue, or a polyamino acid residue.

Further, the groups included in the substituent group T include: a carboxy group; a phosphono group; a sulfo group; an onio group; an amino acid residue; and the above-described alkyl group, alkenyl group, alkynyl group, cycloalkyl group, cycloalkenyl group, aryl group, heterocycle group, alkoxy group, alkenyloxy group, alkynyloxy group, cycloalkyloxy group, aryloxy group, heterocyclic oxy group, alkoxycarbonyl group, cycloalkoxycarbonyl group, aryloxycarbonyl group, amino group, sulfamoyl group, acyl group, acyloxy group, carbamoyl group, acylamino group, sulfonamide group, alkylthio group, cycloalkylthio group, arylthio group, heterocyclic thio group, and an alkyl, cycloalkyl, or aryl sulfonyl group, which have a polyamino acid residue as a substituent.

The substituent selected from the substituent group T is more preferably an alkyl group, an alkenyl group, a cycloalkyl group, an aryl group, a heterocyclic group, an alkoxy group, a cycloalkoxy group, an aryloxy group, an alkoxycarbonyl group, a cycloalkoxycarbonyl group, an amino group, an acylamino group, a cyano group or a halogen atom, and particularly preferably an alkyl group, an alkenyl group, an aryl group, a heterocyclic group, an alkoxy group, an alkoxycarbonyl group, an amino group, an acylamino group, or a cyano group.

The substituent selected from the substituent group T also includes a group obtained by combining a plurality of the above groups, unless otherwise specified. For example, in a case where a compound, a substituent, or the like contains an alkyl group, an alkenyl group, or the like, the alkyl group, the alkenyl group, or the like may be substituted or unsubstituted. In addition, in a case where a compound, a substituent, or the like contains an aryl group, a heterocyclic group, or the like, the aryl group, the heterocyclic group, or the like may be a monocyclic ring or a fused ring, and may be substituted or unsubstituted.

### [Cured product]

The cured product according to the embodiment of the present invention is a cured product obtained by curing the adhesive according to the embodiment of the present invention. That is, the cured product according to the embodiment of the present invention is used as a member constituting an adhesive portion of an endoscope. A curing temperature of the adhesive according to the embodiment of the present invention is not particularly limited. The adhesive according to the embodiment of the present invention can efficiently proceed a curing reaction even in a low temperature range, and can obtain a cured product according to the embodiment of the present invention. It is preferable that the mixing of each component is performed while removing air bubbles, and thus the mixing is usually performed under reduced pressure. The curing temperature is preferably 100°C or lower, more preferably 80°C or lower, still more preferably 60°C or lower, and can also be set to 50°C or lower. In addition, in order to sufficiently perform the curing reaction, the curing temperature is preferably 0°C or higher, and more preferably 10°C or higher. As a preferred range, 0 to 100°C is preferable, 0 to 80°C is more preferable, 10 to 60°C is still more preferable, and 10 to 50°C is particularly preferable. A curing reaction time can be appropriately set according to the purpose. The curing reaction is usually performed for 1.5 to 200 hours to obtain the cured product.

### [Endoscope]

In the endoscope according to the embodiment of the present invention, a constituent member is fixed by the cured product according to the embodiment of the present invention. The phrase "the constituting member is fixed by the cured product according to the embodiment of the present invention" means that at least a part of members constituting the endoscope is fixed to the support member through the cured product according to the embodiment of the present invention.

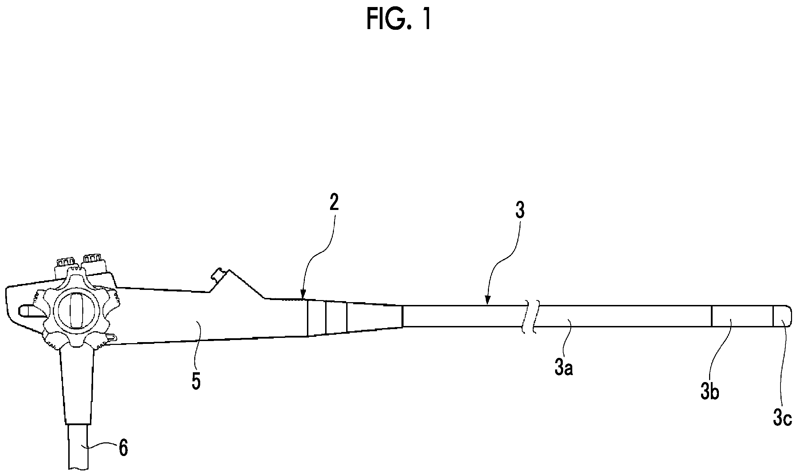

An example of the endoscope (electronic endoscope) according to the embodiment of the present invention will be described. The electronic endoscope is incorporated with a flexible tube for an endoscope (hereinafter, the flexible tube for an endoscope may be simply referred to as a "flexible tube") and is widely used as a medical device. In the example shown in FIG. 1, the electronic endoscope 2 comprises an insertion part 3 to be inserted into a body cavity, a body operation unit 5 connected to a proximal end part of the insertion part 3, and a universal cord 6 connected to a processor device and a light source device. The insertion part 3 is composed of a flexible tube 3a connected to the body operation unit 5, an angle part 3b connected to the flexible tube 3a, and a distal end part 3c connected to a distal end of the angle part 3b, which is mainly composed of a metal (for example, stainless steel) member. An imaging device (not shown) for body cavity imaging is built in the distal end part 3c. The flexible tube 3a, which forms most of a length of the insertion part 3, has flexibility over substantially the entire length thereof, and has a structure in which, in particular, a portion to be inserted into a body cavity or the like is highly flexible.

In FIG. 1, a plurality of channels (tubes, not shown) penetrating in an axial direction of the insertion part 3 are formed in a distal end surface of the distal end part 3c from the body operation unit 5.

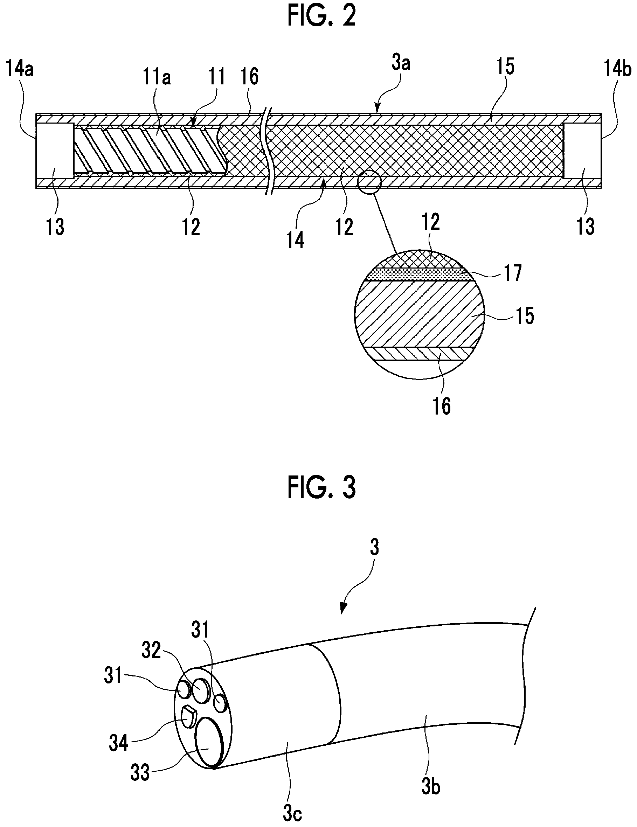

As shown in FIG. 2, the flexible tube 3a in FIG. 1 has a configuration in which a resin layer 15 is coated on an outer peripheral surface of a flexible tube base material 14.

A portion 14a is a distal end side (side of the distal end part 3c), and a portion 14b is a proximal end side (side of the body operation unit 5).

The flexible tube base material 14 is formed by coating a spiral tube 11 formed by spirally winding a metal strip 11a on the innermost side with a cylindrical mesh body 12 formed by braiding metal wires. A ferrule 13 is fitted to each of both ends thereof. The resin layer 15 is bonded to the flexible tube base material 14 via an adhesive cured product layer 17. The adhesive cured product layer 17 can be formed by applying the adhesive according to the embodiment of the present invention and curing the adhesive. The adhesive cured product layer (adhesive portion) 17 is shown as a layer having a uniform thickness for convenience of illustration, but the form is not limited thereto, and the adhesive cured product layer 17 may be irregularly interposed between the resin layer 15 and the flexible tube base material 14. The thickness may be almost zero, and the resin layer 15 and the flexible tube base material 14 may be substantially in contact with each other and bonded to each other.

A coating layer 16 containing, for example, fluorine having chemical resistance is coated on an outer surface of the resin layer 15. It should be noted that the adhesive cured product layer 17, the resin layer 15, and the coating layer 16 are drawn to be thick with respect to a diameter of the flexible tube base material 14 in order to clearly illustrate the layer structure.

As shown in FIG. 3, an illumination window 31, an observation window 32, and a forceps port 33 are formed in the distal end surface of the distal end part 3c. In addition, in order to clean the distal end surface as necessary, a nozzle 34 that sends out water and air is formed. The illumination window 31, the observation window 32, the forceps port 33, and the nozzle 34 are connected to the body operation unit 5 by a channel.

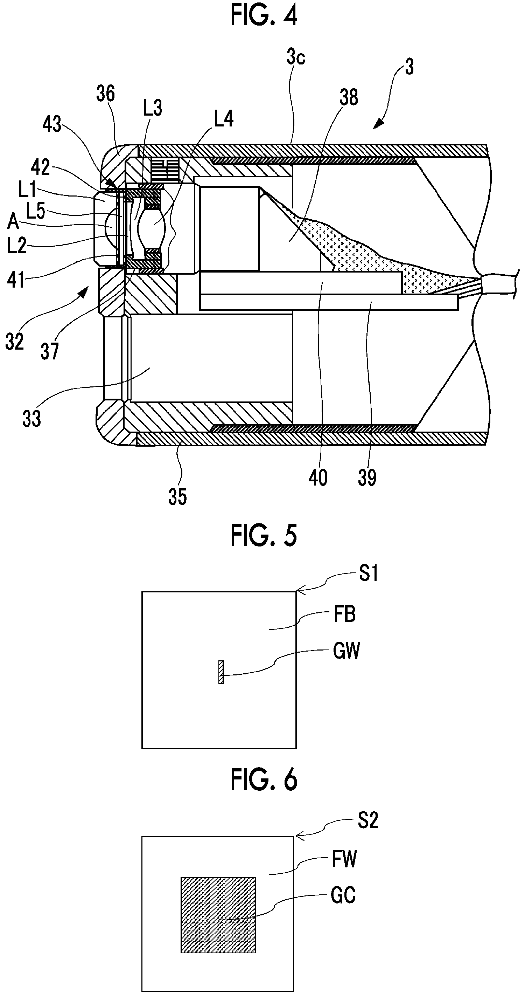

As shown in FIG. 4, the distal end part 3c is composed of a distal end part body 35 made of metal and a distal end cap 36 made of an electrically insulating member.

An observation unit 43 that is an optical system device is installed in the observation window 32. In the observation unit 43, an objective optical system composed of lenses L1 to L5 is fixed in a lens holder 37 by adhesive cured products 41 and 42. The adhesive cured products 41 and 42 can be formed by applying the adhesive according to the embodiment of the present invention and curing the adhesive. In the objective optical system, A is an air layer. A prism 38 is bonded and fixed to an end surface of the lens holder 37. The light axis of the objective optical system is bent at a right angle by the prism 38. The prism 38 is fixed to a solid-state imaging element 40. The solid-state imaging element 40 is fixed to a substrate 39. The adhesive according to the embodiment of the present invention can also be applied to the fixation.

### <Method of manufacturing endoscope>

The method of manufacturing an endoscope according to the embodiment of the present invention is not particularly limited as long as it includes fixing an endoscope constituent member using the adhesive according to the embodiment of the present invention, and the steps other than the fix of the endoscope constituent member can be performed by adopting a normal manufacturing step to manufacture the endoscope according to the embodiment of the present invention.

A material of the fixed endoscope constituent member is not particularly limited, and examples thereof include a resin member, a metal member, and a glass member. The endoscope constituent member can be fixed to a support member constituting the endoscope or the like by, for example, preferably mixing each component included in the adhesive according to the embodiment of the present invention under reduced pressure, applying the mixture to an application site, and heating the mixture at -10°C to 60°C (preferably 0°C to 60°C and more preferably 10°C to 50°C) for 1.5 to 200 hours.

Hereinafter, a use form of the adhesive in the method of manufacturing an endoscope according to the embodiment of the present invention will be described with specific examples, but the present invention is not limited thereto.

Examples of the resin member among the endoscope constituent members fixed by the adhesive according to the embodiment of the present invention include a tube to be inserted into the insertion part of the endoscope. Examples of the resin material constituting the above-described tube include a fluororesin such as Teflon (registered trademark), polysulfone, polyester, polyolefin, and silicone. The adhesive according to the embodiment of the present invention can be used, for example, for bonding (fixing the tube to the metal member or the glass member) between the metal member or the glass member constituting the insertion part of the endoscope and the tube.

In addition, as described above, the adhesive according to the embodiment of the present invention can also be used to form the adhesive cured product layer 17 in FIG. 2. In addition, the adhesive according to the embodiment of the present invention can also be used for bonding the resin layer 15 and the coating layer 16 in FIG. 2.

The adhesive according to the embodiment of the present invention can be used for finishing and fixing an outer surface of an end part (end part on the distal end side (side of the angle part 3b) of the flexible tube 3a) of a flexible outer sheath tube (resin layer 15). Specifically, the end part of the resin layer 15 of the flexible tube 3a is tightly bound with a thread from the outside and fixed to the inner member, and the adhesive is applied to cover the thread and cured. The reason for constituting the outermost layer of the distal end side end part of the flexible tube 3a with the adhesive according to the embodiment of the present invention is to make the thread of the distal end side end part less likely to fray and to make it easier to insert the insertion part into the body cavity.

In addition, the adhesive according to the embodiment of the present invention can be used for at least one of bonding between the distal end part 3c and the angle part 3b or bonding between the insertion part 3 and the body operation unit 5. For example, the distal end part 3c and the angle part 3b are bonded to each other using the adhesive according to the embodiment of the present invention, and then the bonded portion of the distal end part 3c and the angle part 3b and the vicinity thereof are wound and tightened with a thread to reinforce the bonding, and the adhesive is applied to cover the thread and cured. The same applies to the bonding between the insertion part 3 and the body operation unit 5.

In addition, the adhesive according to the embodiment of the present invention can also be used for fixing various tubes to be inserted into the insertion part of the endoscope to at least one of the distal end part 3c or the body operation unit 5.

In addition, it is preferable that the adhesive according to the embodiment of the present invention is also used for sealing (fixing the glass member) of the illumination window 31 and the observation window 32 in the distal end part 3c. By applying the adhesive in a thick manner, the corner portion of the lens outer periphery can be smoothed, and incidence of light from the lateral direction of the lens can be blocked.

In addition, the adhesive according to the embodiment of the present invention can be used for fixing members such as assembling an imaging device built in the distal end part 3c, bonding components, and sealing the solid-state imaging element 40. The imaging device includes an optical system consisting of a plurality of optical components such as the lenses L1 to L5 and the prism 38, and a solid-state imaging element 40 such as a charge coupled device (CCD) that photoelectrically converts an optical image formed by the optical system into an imaging signal. The adhesive according to the embodiment of the present invention can be used for bonding optical components such as the lenses L1 to L5 and the prism 38, which are made of a material such as glass, to each other, and for bonding the lenses L1 to L5 and the prism 38 to the substrate 39 made of a resin or a metal, and the glass member can be fixed and the metal member can be fixed by the bonding.

In addition, the adhesive according to the embodiment of the present invention can be used for bonding and fixing the solid-state imaging element 40 and the substrate 39 and for sealing. By the bonding, a metal member constituting the solid-state imaging element, the substrate, and the like can be fixed.

As described above, the method of manufacturing an endoscope according to the embodiment of the present invention includes a step of fixing an endoscope constituent member using the adhesive according to the embodiment of the present invention. In particular, the adhesive according to the embodiment of the present invention can be suitably used for bonding the distal end cap 36 in the distal end part 3c to the illumination window 31 and the observation window 32 made of a material such as glass and at least any one of the lenses L1 to L5 or the prism 38.

### [Endoscope System]

The endoscope system according to the embodiment of the present invention includes the endoscope according to the embodiment of the present invention and a light source device having a light source for white irradiation light, and can adopt a configuration of a normal endoscope system.

An example of the endoscope system according to the embodiment of the present invention will be described. The endoscope system according to the embodiment of the present invention includes the endoscope according to the embodiment of the present invention, a light source device having a light source for white irradiation light, a processor device, a display, an operation input unit, and a universal cord. The endoscope images an observation target in a living body. The light source device supplies white illumination light for irradiating the observation target to the endoscope. The processor device functions as a central control unit that performs control related to imaging, illumination light, or the like, and also processes an image signal obtained through imaging to generate a display image or the like. The processor device is electrically connected to the display and the operation input unit. The display displays an image captured by the endoscope and image information and the like associated with the image. The operation input unit functions as a user interface that receives input operations such as function settings. The universal cord is a cord through which a communication cable extending from an insertion part, a light guide, or the like is inserted, and connects the endoscope to the processor device and the light source device. Therefore, the endoscope has the light guide that guides illumination light. It should be noted that an external recording unit that records an image, image information, and the like may be connected to the processor device.

The endoscope is optically connected to the light source device and is electrically connected to the processor device. The endoscope includes an insertion part to be inserted into a subject, and an operation part provided at a proximal end part of the insertion part, and has a bendable part and a distal end part provided on a distal end side of the insertion part. By operating an angle knob of the operation part, the bendable part performs a bending operation. By this bending operation, the distal end is directed in a desired direction. In addition, the operation part is provided with a zoom operation part and the like in addition to the angle knob.

In the endoscope system according to the embodiment of the present invention, observation (white light observation) using the white irradiation light supplied from the light source device is performed.

The light source included in the light source device can be used without particular limitation as long as the light source can supply the white illumination light to the endoscope, and for example, a white light source such as a halogen lamp or a xenon lamp can be used.

In addition, the white irradiation light may be generated by controlling the amount of each color of light emitted from each of the plurality of semiconductor light sources (preferably, light emitting diode (LED) displays) and combining the each color of light by a multiplexer member such as a dichroic filter. The multiplexer member is an optical element having a wavelength selection function, that is, an optical element having a function of reflecting light in a specific wavelength range and transmitting light in a specific wavelength range in a case where light is incident on the multiplexer member. Even in a case where each of the plurality of semiconductor light sources emits light in a different direction, the white irradiation light can be generated by combining the light and causing the light to be incident on the condenser lens by the wavelength selection function of each multiplexer member and the arrangement and the like of each multiplexer member.

As the light source device that generates the white irradiation light using the plurality of semiconductor light sources, the description related to the light source device for an endoscope that generates the irradiation light of the white light, which is described in JP2013-109319A, can be applied.

Examples of the light source device for an endoscope that generates the white irradiation light by combining the light of each color using the plurality of semiconductor light sources include a light source device including a plurality of first semiconductor light sources that emit light in different wavelength ranges, an illumination light generation unit that generates the illumination light of the white light by the light emitted from the plurality of first semiconductor light sources, and a light source attachment unit that is attachably and detachably configured with a second semiconductor light source, in which the illumination light generation unit generates the white irradiation light by the light emitted from the plurality of first semiconductor light sources and the light emitted from the second semiconductor light source in a case where the second semiconductor light source is attached to the light source attachment unit.

The illumination light generation unit includes a multiplexing member, a collimator lens, and a condenser lens.

The multiplexer member is preferably a dichroic filter, and the dichroic filter has a characteristic of transmitting light in a specific wavelength range and reflecting light in a specific wavelength range such that the light emitted from each of the plurality of first semiconductor light sources is incident on the condenser lens to generate the illumination light.

The collimator lens adjusts the light emitted from each semiconductor light source to parallel light.

The condenser lens generates and emits the illumination light of the white light by the light emitted from each semiconductor light source incident thereon.

In the light source device that generates the white irradiation light by combining the light as described above, preferred specific examples of the semiconductor light source include any one of the following forms 1) to 10) or a form in which two or more of the following forms 1) to 10) are combined.
1) Each of the first semiconductor light sources emits any one piece of light selected from among violet light, blue light, green light, and red light, the plurality of first semiconductor light sources include at least three first semiconductor light sources, and a continuous wavelength range is formed by light emitted from each of the plurality of first semiconductor light sources.
   As the semiconductor light source that emits violet light, a violet LED is preferably used, as the semiconductor light source that emits blue light, a blue LED is preferably used, as the semiconductor light source that emits green light, a green LED is preferably used, and as the semiconductor light source that emits red light, a red LED is preferably used. The same applies to the following 2) to 10).
2) The plurality of first semiconductor light sources include at least four first semiconductor light sources that each emit any one piece of light selected from among violet light, blue light, green light, and red light, and a continuous wavelength range is formed by using light emitted from each of the plurality of first semiconductor light sources.
3) In a case where light emitted from each of two first semiconductor light sources among the plurality of first semiconductor light sources forms a continuous wavelength range, a wavelength range of the light emitted from the second semiconductor light source includes a wavelength range between the wavelength ranges of the pieces of light emitted from the two first semiconductor light sources.
   As the second semiconductor light source, for example, a cyan LED that emits cyan light is preferably used. The same applies to the following 4) to 10).
4) The plurality of first semiconductor light sources include the first semiconductor light source that emits blue light and the first semiconductor light source that emits green light, and a wavelength range of the light emitted from the second semiconductor light source includes a wavelength range between a wavelength range of the blue light emitted from the first semiconductor light source and a wavelength range of the green light emitted from the first semiconductor light source.
5) The plurality of first semiconductor light sources include the first semiconductor light source that emits green light and the first semiconductor light source that emits red light, and a wavelength range of the light emitted from the second semiconductor light source includes a wavelength range between a wavelength range of the green light emitted from the first semiconductor light source and a wavelength range of the red light emitted from the first semiconductor light source.
6) The plurality of first semiconductor light sources include the first semiconductor light source that emits violet light and the first semiconductor light source that emits blue light, and a wavelength range of the light emitted from the second semiconductor light source includes a wavelength range between a wavelength range of the violet light emitted from the first semiconductor light source and a wavelength range of the blue light emitted from the first semiconductor light source.
7) The wavelength range of the light emitted from the second semiconductor light source includes a wavelength range of a long wavelength range with respect to the wavelength range of the light emitted from the plurality of first semiconductor light sources.
8) The wavelength range of the light emitted from the second semiconductor light source includes a wavelength range of a near-infrared range.
9) The wavelength range of the light emitted from the second semiconductor light source includes a wavelength range of a short wavelength range with respect to the wavelength range of the light emitted from the plurality of first semiconductor light sources.
10) The wavelength range of the light emitted from the second semiconductor light source includes a wavelength range of an ultraviolet range.

In addition, the description related to the endoscope system and the light source for supplying the white irradiation light to the endoscope, which is described in JP2013-109319A, can be preferably applied to the present invention.

### Examples

The present invention will be described in more detail based on Examples, but the present invention is not limited to the following Examples. The blending amount of the component means the blending amount of the component itself. That is, in a case where the raw material includes a solvent, the amount is an amount excluding the solvent.

### [Preparation Example 1] Preparation of adhesive 1

The following components were mixed at the following blending ratio: 100 parts by mass of bisphenol A diglycidyl ether (trade name: jER828, manufactured by Mitsubishi Chemical Corporation, epoxy equivalent: 190) as a component (A); 40 parts by mass of polyoxypropylenediamine (trade name: JEFFAMINE D-230, manufactured by HUNTSMAN Corporation, active hydrogen equivalent: 60) as a component (B); a fluorescent organic compound shown in Table 1 in the amount shown in Table 1; 25 parts by mass of an inorganic amphoteric ion exchanger (trade name: IXEPLAS-B1, manufactured by Toagosei Co., Ltd., an inorganic compound containing Bi atoms and Zr atoms) as a component (D); 10 parts by mass of silica (trade name: AEROSIL NAX50, manufactured by Nippon Aerosil Co., Ltd., surface-modified with a trimethylsilyl group, volume-based median diameter (D50): 30 nm); and 5 parts by mass of carbon black (trade name: DENKA BLACK, manufactured by Denka Company Limited, volume-based median diameter (D50): 35 nm). The mixture was defoamed for 5 minutes while being stirred at 2,000 rpm in a state of being reduced in pressure to 1.0 Pa at 25°C with "THINKY Mixer ARV-310 (trade name, manufactured by THINKY Corporation)", thereby obtaining an adhesive.

### [Preparation Example 2] Preparation of adhesive 2

The following components were mixed at the following blending ratio: 100 parts by mass of a bisphenol A diglycidyl ether (trade name: jER828, manufactured by Mitsubishi Chemical Corporation, epoxy equivalent: 190) as the component (A), a curing agent shown in Table 2 at the parts by mass shown in Table 2, 0.020 parts by mass of 2,5-bis(5-tert-butyl-2-benzoxazolyl)thiophene (the fluorescent organic compound used as No. 102 in Table 1) as the component (C), 25 parts by mass of an inorganic amphoteric ion exchanger (trade name: IXEPLAS-B1, manufactured by Toagosei Co., Ltd.) as the component (D), 10 parts by mass of silica (trade name: AEROSIL NAX50, manufactured by Nippon Aerosil Co., Ltd., surface-modified with a trimethylsilyl group, volume-based median diameter (D50): 30 nm), and 5 parts by mass of carbon black (trade name: DENKA BLACK, manufactured by Denka Company Limited, volume-based median diameter (D50): 35 nm).

The obtained mixture was stirred and defoamed under reduced pressure by the method described in Preparation Example 1 to obtain an adhesive.

In Tables 1 and 2, No. 101 to 110 are the adhesives for an endoscope according to the embodiment of the present invention, and No. c11 to c13 are adhesives for an endoscope for comparison.

The following evaluations were performed using these adhesives for an endoscope. The obtained evaluation results are summarized in Tables 1 and 2.

### [Evaluation 1: Ease of determination of presence or absence of insufficient entry of adhesive into gap]

A rectangular fine hole (depression of the size of GW in FIG. 5) having a width (horizontal) of 0.2 mm, a length (vertical) of 2 mm, and a depth of 2 mm was produced at a center portion of one surface of a black fluororubber plate (thickness: 3 mm, length: 20 mm, width: 20 mm, manufactured by MISUMI Corporation) FB. The adhesive prepared above was poured into the hole using a needle to fill the hole and cure the adhesive, thereby producing an evaluation sample S1 shown in FIG. 5. The evaluation sample S1 was irradiated with a UV black light (manufactured by ICHINEN TASCO Co., Ltd., light source: UV-LED manufactured by NICHIA CORPORATION, 3 lamps), and it was determined whether or not a portion (cured product of adhesive coating material) filled with the adhesive (GW) could be visually recognized.

The above-described determination was performed on 100 samples, and the proportion of samples in which the portion GW filled with the adhesive could be visually recognized was evaluated according to the following evaluation standard to evaluate the ease of determination of the presence or absence of the insufficient entry of the adhesive into the gap.

### - Evaluation standards -

A: 99% or more
B: 95% or more and less than 99%
C: 90% or more and less than 95%
D: 85% or more and less than 90%
E: less than 85%

### [Evaluation 2: Influence on observation image in white light observation]

The adhesive prepared above was applied to a center portion of one surface of a white fluororubber plate (thickness: 3 mm, length: 20 mm, width: 20 mm, manufactured by MISUMI Corporation) FW in a range of 10 mm in length × 10 mm in width (range of GC in FIG. 6), and cured to produce an evaluation sample S2 shown in FIG. 6. The obtained evaluation sample S2 was observed with white light using an endoscope (manufactured by FUJIFILM Corporation, trade name: EG-6600R) in an environment of a dark room, and it was determined whether or not it was possible to visually recognize that the periphery of the cured product GW of the adhesive coating material was illuminated on the observation screen.

The above-described determination was performed on 100 samples, and the proportion of samples in which it was possible to visually recognize that the periphery of the cured product GW of the adhesive coating material was illuminated was evaluated according to the following evaluation standard to evaluate the influence on the observation image in white light observation.

### - Evaluation standards -

A: less than 1%
B: 1% or more and less than 3%
C: 3% or more and less than 5%
D: 5% or more and less than 7%
E: 7% or more

**[Table 1]**

| No. | Fluorescent organic compound | | | | Ease of determination of presence or absence of insufficient entry of adhesive into gap | Influence on observation image in white light observation |
|---|---|---|---|---|---|---|
| | Type | Molecular weight | Maximum fluorescence wavelength | Blending amount | | |
| 101 | 7-(diethylamino)-4-methylcoumarin | 231 | 450 | 0.01 | B | A |
| | | | | 0.1 | A | A |
| | | | | 0.2 | A | A |
| 102 | 2,5-bis(5-tert-butyl-2-benzoxazolyl)thiophene | 430 | 440 | 0.01 | A | A |
| | | | | 0.1 | A | A |
| | | | | 0.2 | A | A |
| 103 | 4,4'-bis(2-benzoxazolyl)stilbene | 414 | 435 | 0.01 | A | A |
| | | | | 0.1 | A | A |
| | | | | 0.2 | A | A |
| 104 | 1,2-bis(5-methyl-2-benzoxazolyl)ethylene | 290 | 420 | 0.01 | A | A |
| | | | | 0.1 | A | A |
| | | | | 0.2 | A | A |
| 105 | Fluorescein | 332 | 521 | 0.01 | A | A |
| | | | | 0.1 | A | B |
| | | | | 0.2 | A | B |
| 106 | Fluorescent organic compound for coating | Unknown | 500 | 0.01 | A | A |
| | | | | 0.1 | A | A |
| | | | | 0.2 | A | B |
| 107 | 7-(dimethylamino)-4-methylcoumarin | 203 | 440 | 0.2 | C | B |
| 108 | 4-methylumbelliferone | 176 | 450 | 0.2 | C | C |
| c11 | Rhodamine 6G | 479 | 551 | 0.01 | E | D |
| | | | | 0.1 | E | D |
| | | | | 0.2 | D | E |
| c12 | Sulforhodamine B | 559 | 585 | 0.01 | E | C |
| | | | | 0.1 | E | D |
| | | | | 0.2 | D | E |

**[Table 2]**

| No. | Curing agent | | Ease of determination of presence or absence of insufficient entry of adhesive into gap | Influence on observation image in white light observation |
|---|---|---|---|---|
| | Type | Blending amount | | |
| 110 | JEFFAMIN D-230 | 32 | A | A |
| 109 | Gaskamine 328 | 29 | C | B |
| cl3 | 2-ethyl-4-methylimidazole | 10 | E | B |

### <Note in Table>

Maximum fluorescence wavelength: maximum fluorescence wavelength in a DMF solution obtained by the above-described measurement method using a spectrophotofluorometer RF-6000 (trade name, manufactured by Shimadzu Corporation), unit: nm

Blending amount: blending amount with respect to 100 parts by mass of bisphenol A diglycidyl ether (trade name: jER828, manufactured by Mitsubishi Chemical Corporation, epoxy equivalent: 190) as the component (A), unit: parts by mass. It is noted that 0.01 parts by mass means 0.010 parts by mass, 0.1 parts by mass means 0.100 parts by mass, and 0.2 parts by mass means 0.200 parts by mass.

### [Fluorescent organic compound]

### 7-(diethylamino)-4-methylcoumarin

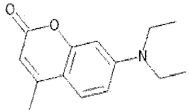

### 2,5-bis(5-tert-butyl-2-benzoxazolyl)thiophene

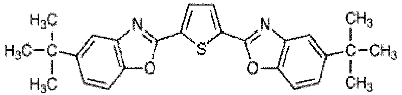

### 4,4'-bis(2-benzoxazolyl)stilbene

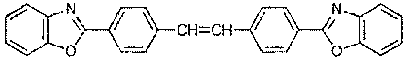

### 1,2-bis(5-methyl-2-benzoxazolyl)ethylene

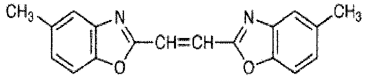

### Fluorescein

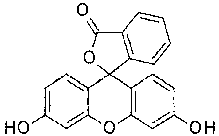

### 7-(dimethylamino)-4-methylcoumarin

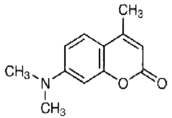

### 4-methylumbelliferone

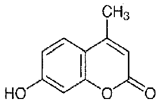

### [Curing agent]

JEFFAMINE D-230: trade name, manufactured by HUNTSMAN Corporation, active hydrogen equivalent: 60

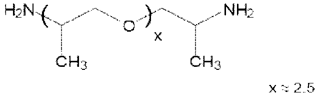

Gaskamine 328: trade name, manufactured by Mitsubishi Gas Chemical Company, Inc., active hydrogen equivalent: 55

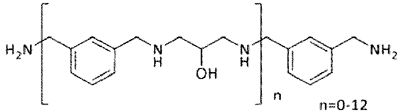

### 2-ethyl-4-methylimidazole

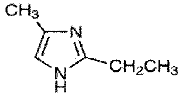

From the results of Tables 1 and 2, the following can be seen.

The adhesives of No. c11 and c12 are not the adhesives according to the embodiment of the present invention in that the fluorescent organic compound having a maximum fluorescence wavelength of more than 525 nm in an N,N-dimethylformamide solution is contained as the fluorescent organic compound. In the adhesives of No. c11 and c12, in any case of 0.01 parts by mass, 0.1 parts by mass, and 0.2 parts by mass, which are considered to be a small amount that does not affect the observation using the endoscope in a case of adding the fluorescent organic compound, the content of the fluorescent organic compound with respect to 100 parts by mass of the epoxy resin of the component (A) was set, and it was not possible to easily determine the presence or absence of the insufficient entry of the adhesive into the gap. Moreover, as the addition amount of the fluorescent organic compound increased, the influence of the adhesive component on the observation image in white light observation using the endoscope increased, and even in a case where the addition amount was increased to 0.2 parts by mass, it was not possible to easily determine the presence or absence of the insufficient entry of the adhesive into the gap, and the influence of the adhesive component on the observation image in white light observation using the endoscope occurred at a rate of 7%.

In addition, the adhesive of No. c13 is not the adhesive according to the embodiment of the present invention in that the curing agent having no unsubstituted amino group is contained as the curing agent of the epoxy resin of the component (A). In the adhesive of No. c13, even in a case where the content of the fluorescent organic compound with respect to 100 parts by mass of the epoxy resin of the component (A) was increased to 0.2 parts by mass, it was not possible to easily determine the presence or absence of the insufficient entry of the adhesive into the gap.

On the other hand, in the adhesives of No. 101 to 108, which are the adhesives according to the embodiment of the present invention, in any case of 0.01 parts by mass, 0.1 parts by mass, and 0.2 parts by mass, the content of the fluorescent organic compound with respect to 100 parts by mass of the epoxy resin of the component (A) was set, and it was possible to easily determine the presence or absence of the insufficient entry of the adhesive into the gap, and it was possible to suppress the influence of the adhesive component on the observation image in white light observation using the endoscope. In addition, in the adhesives of No. 109 and 110, which are the adhesives according to the embodiment of the present invention, in a case where the fluorescent organic compound was blended at 0.2 parts by mass with respect to 100 parts by mass of the epoxy resin of the component (A), it was possible to easily determine the presence or absence of the insufficient entry of the adhesive into the gap, and it was possible to suppress the influence of the adhesive component on the observation image in white light observation using the endoscope. In particular, the adhesive of No. 110, which includes the polyether polyamine compound as the curing agent of the epoxy resin of the component (A), was excellent in that it was possible to more easily determine the presence or absence of the insufficient entry of the adhesive into the gap and it was possible to further suppress the influence of the adhesive component on the observation image in white light observation using the endoscope, as compared with the adhesive of No. 109, which does not include the polyether polyamine compound as the curing agent of the epoxy resin of the component (A).

The present application claims priority based on JP2023-170304 filed on September 29, 2023, the entire content of which is incorporated herein by reference.

### Explanation of References

2: electronic endoscope (endoscope)
3: insertion part
3a: flexible tube
3b: angle part
3c: distal end part
5: body operation unit
6: universal cord
11: spiral tube
11a: metal strip
12: cylindrical mesh body
13: ferrule
14: flexible tube base material
14a: distal end side
14b: proximal end side
15: resin layer
16: coating layer
17: adhesive cured product layer
31: illumination window
32: observation window
33: forceps port
34: nozzle
35: distal end part body
36: distal end cap
37: lens holder
38: prism
39: substrate
40: solid-state imaging element
41: adhesive cured product
42: adhesive cured product
43: observation unit
A: air layer
L1 to L5: lens
FB: black fluororubber plate
FW: white fluororubber plate
GW: cured product of adhesive that fills hole portion of fluororubber plate
GC: cured product of adhesive coating material
S1: sample for evaluation
S2: sample for evaluation

## Claims

1. An adhesive for an endoscope, comprising the following components (A) to (C):
(A) an epoxy resin;
(B) a polyamine compound having two or more unsubstituted amino groups; and
(C) a fluorescent organic compound having a maximum fluorescence wavelength of 380 to 525 nm in an N,N-dimethylformamide solution.

2. The adhesive for an endoscope according to claim 1,
wherein the component (B) includes a polyether polyamine compound.

3. The adhesive for an endoscope according to claim 1,
wherein a content of the component (C) is 0.005 to 0.500 parts by mass with respect to 100 parts by mass of the component (A).

4. The adhesive for an endoscope according to claim 1, further comprising:
5 to 30 parts by mass of an inorganic amphoteric ion exchanger as a component (D) with respect to 100 parts by mass of the component (A).

5. An endoscope comprising:
a member fixed by the adhesive for an endoscope according to any one of claims 1 to 4.

6. A manufacturing method of an endoscope, comprising:
fixing a member by using the adhesive for an endoscope according to any one of claims 1 to 4.
